# EUROPEAN PATENT APPLICATION

(11) **EP 3 462 460 A1**
(43) Date of publication of application: **03.04.2019**
(21) Application number: 17193421.9
(22) Date of filing: 27.09.2017
(51) Int. Cl.: G16H 40/20, G06Q 10/06, G06Q 50/00

(54) **METHOD AND SYSTEM FOR ALLOCATING MEDICAL DATA RECORD TO HUMAN RESOURCE DATA RECORD**

(71) Applicant: Siemens Healthcare GmbH, 91052 Erlangen (DE)
(72) Inventor: Athmanathan, Balakrishnan, 560061 Bangalore (IN); Rangapura Shettappa, Chandrashekara, 560100 Bengaluru (IN); Thomas, Arun, 691507 Kollam, Kerala (IN)
(74) Representative: Patentanwälte Bals & Vogel

(57) **Abstract**

A method and a system for allocating a medical data record to a human resource data record are disclosed. In one aspect of the invention, the method includes receiving (301) a medical data record from a source (103) by an interface (207) and receiving (301) a set of human resource data records from a second source (104) by the interface (207). In another aspect of the invention, the method includes analyzing (302) the medical data record by a processing unit (201) to identify a parameter of the medical data record. In yet another aspect of the invention, the method includes determining (303) a preference for each one of the set of human resource data records by the processing unit (201); and determining (304) a matching human resource data record from the set of human resource data records based on the preference and the parameter of the medical data record by the processing unit (201); In a further aspect of the invention, the method includes allocating (305) the medical data record to the matched human resource data record by the processing unit (201).

## Description

The invention relates to a method of allocating medical data record to a human resource data record. The method and system disclosed therein comprises the steps of receiving medical data record from a source; analyzing the received medical data to identify a parameter of the medical data record; and allocating the medical data record to a human resource. The invention also relates to a system or a local transmission unit for allocating medical data record to a human resource data record. The local transmission unit disclosed therein comprises a processing unit configured to perform the method steps of receiving medical data from a source; analyzing the received medical data to identify at least one or more parameters of the medical data; and allocating the medical data to at least one human resource.

Such a method and system have already been disclosed in US 2009/0048866 A1. The method and system disclosed in the aforementioned patent document includes receiving, through a processing unit, a report comprising medical data. The report is analyzed to determine parameters of routing the medical data to a concerned human resource. Based on the parameters, the medical data is transmitted or allocated to the concerned human resource.

However, such allocation of data to one or more human resources may not be dynamic. Each human resource may have certain domain expertise and preference for evaluation and analysis of such medical data. If a medical data is allocated to a human resource who has no/insufficient domain expertise, the medical data may not be analyzed properly, or may be analyzed incorrectly. There may also be cases where a human resource may end up analyzing a medical data which he/she has least priority to analyze. Therefore, such human resource may end up not receiving cases that may be of higher priority. Also, if a medical data is allocated to a human resource without taking into consideration the schedule of such human resource, there may be a delay in receiving the analysis of such medical data. This may cause unwanted delay in administering right treatment to a patient, thereby increasing the risk to health and life of the patient.

Therefore, there exists a need for a method and system which allocates medical data record to human resources dynamically. There also exists a need for a method and system which take into consideration the expertise, availability and preferences of human resources for allocation of medical data record for further analysis.

The object of the invention is therefore to provide a dynamic and flexible way to allocate medical data record to a human resource data record.

This object is solved by a method of allocation of medical data record to a human resource data record according to claim 1, a local transmission unit according to claim 12, a computer program product according to claim 14 and a computer-readable medium according to claim 15.

In the following, the solution according to the invention is described with respect to the claimed local transmission unit as well as with respect to the claimed method. Features, advantages or alternative embodiments herein can be assigned to the other claimed objects and vice versa. In other words, claims for the local transmission unit can be improved with features described or claimed in the context of the method. In this case, the functional features of the method are embodied by objective units of the local transmission unit.

Based on the aforementioned method, the invention achieves the object by determining a preference from the human resource; determining a matching human resource data record based on the preference and identified parameter of the medical data record; and allocating the medical data record to the human resource data record of the matching human resource. An advantage of the invention is that as the medical data record is allocated to a human resource data record based on the defined preference. Therefore, the analysis of the medical data record is more efficient. Furthermore, undue delay in examination of the medical data record is avoided. By allocating the medical data record according to the defined preferences, the medical data record can be analyzed by a human resource having necessary expertise in the corresponding medical domain.

The present invention describes a method of allocating medical data record to a human resource data record. The method comprises a step of receiving medical data record from a source and receiving a set of human resource data records from a second source, by an interface. The source may be, for example, a medical database. The medical database may contain medical data obtained from, but not limited to, one or more medical equipments, for example scanners; hospitals; medical professionals; and other providers that may be involved in patient care. The second source may be, for example, a human resource preference database. The human resource data records contain details of corresponding human resources and may include preferences of the human resources with respect to their interest for analysis of medical data record. The interface may be for example, a data bus, which is configured to receive data from the medical database and the human resource preference database. The received medical data record is analyzed by a processing unit to identify a parameter of the medical data record. Analysis of the medical data record enables identification of one or more medical conditions that a patient may have. Based on the analysis, the medical data record is allocated to a human resource data record for further analysis. In a preferred embodiment, the method further comprises determining by the processing unit a preference for each one of the set of human resource data records. In an embodiment, the human resources may be queried by the processing unit to obtain their preferences on the type of medical data record that may interest the human resources. The obtained preferences may be stored in the human resource preference database. The preferences are used to determine a matching human resource data record and the medical data record is allocated by the processing unit to the matching human resource data record. The advantage of allocating medical data record to a human resource based on set preferences is that the medical data record would be analyzed by an appropriate human resource. Therefore, the risk of delayed analysis or incorrect analysis is eliminated.

According to an embodiment of the invention, the preference includes a determinant defined by a human resource for allocation of the medical data record to the human resource data record. The determinant may be obtained from the human resource by querying the human resource to determine the interest of the human resource with respect to analysis of the medical data records.

According to an embodiment of the invention, the preference is chosen from a group comprising area of specialization/expertise of the human resource, availability of the human resource, and area of interest of the human resource. Therefore, efficient allocation of the medical data record is ensured depending upon the availability of the human resource, area of expertise of the human resource and the area of interest of the human resource.

According to an embodiment of the invention, the medical data record comprises a first data item and a second data item. The first data item includes personal data related to a patient. For example, the first data item may include a patient's name, sex, and contact information. The second data item comprises patient diagnosis information.

According to an embodiment of the invention, in analyzing the medical data record, the method further comprises extracting the second data item from the medical data record and determining from the second data item the medical parameter by the processing unit.

According to an embodiment of the invention, the medical parameters of the medical data record may include patient diagnosis information such as, but not limited to, medical examination history, medical treatment and/or referrals documented in the medical data record. The medical examination history may include, for example, medical image data, results of medical tests, or any other data collected from examination of a patient. The referrals in the medical data record include any recommendation made by a human resource who may have previously examined the medical data record. Advantageously, extracting the second data item from the medical data record enables segregation of medical diagnosis information from patient personal information. The second data item contains details necessary for determination of a matching human resource data record for analysis of the medical data record. The determination of the matching human resource data record is performed essentially based on the medical parameters present in the second data item.

According to an embodiment of the invention, in determining a matching human resource data record, the method further comprises determining from the second data item whether a human resource has been specified in the medical data record, by the processing unit. The medical data record may contain referrals including recommendations made by a human resource who may have examined the medical data record in the past. The recommendations may include a human resource who may be eligible to further analyze the medical data record. Such recommendations are analyzed to determine if a human resource has been specified in the medical data record. If a human resource has been specified, the medical data record is allocated by the processing unit to the human resource data record of the specified human resource. If no human resource has been specified, a matching human resource data record is determined by the processing unit. Advantageously, the medical data record may be allocated to a human resource data record of a human resource if such human resource has been specified already in the medical data record. Therefore, in cases where such human resource has already been specified, no determination of a matching human resource data record may be required, which expedites the execution of the method.

According to an embodiment of the invention, in determining a matching human resource data record, the method further comprises analyzing by the processing unit the preference determined for each one of the set of human resources data records to determine a human resource whose preference matches with the medical parameter. The medical parameters obtained from the medical data record are used for determining a matching human resource from the human resource preference database. The medical parameter is matched with the preferences stored in the human resource preference database. When a matching human resource is determined, the medical data record is allocated to the human resource data record of the matched human resource. The advantage of determining a matching human resource is to enable the allocation of medical data record to a human resource who would be capable of analyzing the medical data correctly and efficiently.

According to a preferred embodiment of the invention, the method further comprises encrypting the medical data record before allocating the medical data record to the human resource data record. Encryption of medical data record ensures secure transfer of medical data record to the human resource. The encryption may be performed using methods that may be known to a person skilled in the art.

According to an embodiment of the invention, the method further comprises receiving a request, from a server, to access the encrypted medical data record. A human resource may be required to obtain access to the medical data record so as initiate analysis. Therefore, a request to access the medical data may be made by the human resource. On obtaining such a request from the server, the request is authenticated by the processing unit to identify if the request has been received from an authentic human resource. On successful authentication, the medical data record is shared with the authenticated human resource. In an embodiment, such a request may be received from a matched human resource once a medical data record is allocated to the matched human resource data record. This step enables maintenance of security of medical data record. In an alternate embodiment, the matched human resource may choose to perform his/her analysis on one computing unit. Any change made to the medical data record may be stored in an encrypted format in the medical database. Therefore, the state of the medical data record is maintained. The human resource may further choose to transfer the medical data record to a second computing unit, for the sake of convenience, and continue the analysis. When the human resource attempts to access the medical data record on the second computing unit, the step of authentication may be performed again. Therefore, advantageously, security of the medical data record is maintained.

According to another embodiment of the invention, the human resource can share the medical data record with one or more human resources. Therefore, expert advice from one or more human resources may be obtained on a single medical data record. Such sharing of medical data record from one human resource to another is enabled through a secure server and may involve authentication of one or more human resources.

The invention also relates to a local transmission unit configured for allocating medical data record to a human resource data record. The local transmission unit comprises an interface configured to receive a medical data record from a source. The source may be, for example a medical database. The medical database may contain medical data obtained from, but not limited to, one or more medical equipments, for example scanners, hospitals, medical professionals, and other providers that may be involved in patient care. The interface is further configured to receive a set of human resource data records from a second source. The second source may be, for example, a human resource preference database. The human resource data records contain details of corresponding human resources and may include preferences of the human resources with respect to their interest for analysis of medical data record. The interface may be for example, a data bus, which is configured to receive data from the medical database and the human resource preference database. The local transmission unit further comprises a processing unit configured to analyze the received medical data to identify one or more parameters of the medical data. Analysis of the medical data enables identification of one or more medical conditions that a patient may have. The processing unit further is configured to determine a preference for each one of the set of human resource data records. In an embodiment, the human resources may be queried by the processing unit to obtain their preferences on the type of medical data record that may interest the human resources. The obtained preferences may be stored, for example, in the human resource preference database. The processing unit is further configured to use the preferences to determine a matching human resource data record and allocate the medical data record to the human resource data record of the matching human resource. The advantage of allocating medical data to a human resource based on set preferences is that the medical data would be analyzed by an appropriate human resource. Therefore, the risk of delayed analysis or incorrect analysis is eliminated.

According to an embodiment of the invention, the preference includes a determinant defined by a human resource for allocation of the medical data record to the human resource data record. The determinant may be obtained from the human resource by querying the human resource to determine the interest of the human resource with respect to analysis of the medical data records.

According to an embodiment of the invention, the preference is chosen from a group comprising area of specialization/expertise of the human resource, availability of the human resource, and area of interest of the human resource. Therefore, efficient allocation of the medical data record is ensured depending upon the availability of the human resource, area of expertise of the human resource and the area of interest of the human resource.

According to an embodiment of the invention, the medical data record comprises a first data item and a second data item. The first data item includes personal data related to a patient. For example, the first data item may include a patient's name, sex, and contact information. The second data item comprises patient diagnosis information.

According to an embodiment of the invention, in analyzing the medical data, the processing unit is further configured to extract the second data item from the medical data record and determine from the second data item the medical parameter. According to an embodiment of the invention, the medical parameters of the medical data record may include patient diagnosis information such as medical examination history, medical treatment and/or referrals documented in the medical data record. The medical examination history may include, for example, medical image data, results of medical tests, or any other data collected from examination of a patient. The referrals in the medical data record include any recommendation made by a human resource who may have previously examined the medical data record. Advantageously, extracting the second data item from the medical data record enables segregation of medical diagnosis information from patient personal information. The second data item contains details necessary for determination of a matching human resource data record for analysis of the medical data record. The determination of the matching human resource data record is performed essentially based on the medical parameters present in the second data item.

According to an embodiment of the invention, in determining a matching human resource data record, the processing unit is further configured to determine from the second data item whether a human resource has been specified in the medical data record, by the processing unit. The medical data record may contain referrals including recommendations made by a human resource who may have examined the medical data record in the past. The recommendations may include a human resource who may be eligible to further analyze the medical data record. Such recommendations are analyzed to determine if a human resource has been specified in the medical data record. If a human resource has been specified, the medical data record is allocated by the processing unit to the human resource data record of the specified human resource. If no human resource has been specified, a matching human resource data record is determined by the processing unit. Advantageously, the medical data record may be allocated to a human resource data record of a human resource if such human resource has been specified already in the medical data record. Therefore, in cases where such human resource has already been specified, no determination of a matching human resource data record may be required, which expedites the execution of the method.

According to an embodiment of the invention, in determining a matching human resource data record, the processing unit is further configured to analyze the preference determined for each one of the set of human resources data records to determine a human resource whose preference matches with the medical parameter. The medical parameters obtained from the medical data record are used for determining a matching human resource from the human resource preference database. The medical parameter is matched with the preferences stored in the human resource preference database. When a matching human resource is determined, the medical data record is allocated by the processing unit to the human resource data record of the matched human resource. The advantage of determining a matching human resource is to enable the allocation of medical data record to a human resource who would be capable of analyzing the medical data correctly and efficiently.

According to a preferred embodiment of the invention, the processing unit is further configured to encrypt the medical data record before allocating the medical data record to the human resource data record. Encryption of medical data record ensures secure transfer of medical data record to the human resource. The encryption may be performed using methods that may be known to a person skilled in the art.

According to an embodiment of the invention, the processing unit is configured to receive a request, from a server, to access the encrypted medical data record. A human resource may be required to obtain access to the medical data record so as initiate analysis. Therefore, a request to access the medical data record may be made by the human resource. On obtaining such a request from the server, the processing unit is further configured to authenticate the request to identify if the request has been received from an authentic human resource. On successful authentication, the processing unit is further configured to share the medical data record with the authenticated human resource. In an embodiment, such a request may be received from a matched human resource once a medical data record is allocated to the matched human resource. This step enables maintenance of security of the medical data record. In an alternate embodiment, the matched human resource may choose to perform his/her analysis on one computing unit. Any change made to the medical data record may be stored in an encrypted format in the medical database. Therefore, the state of the medical data record is maintained. The human resource may further choose to transfer the medical data record to a second computing unit, for the sake of convenience, and continue the analysis. When the human resource attempts to access the medical data record on the second computing unit, the step of authentication may be performed again. Therefore, advantageously, security of the medical data record is maintained.

The invention relates in one aspect to a computer program product comprising a computer program, the computer program being loadable into a storage unit of a local transmission unit, including program code sections to make the local transmission unit execute the method according to an aspect of the invention when the computer program is executed in the local transmission unit.

The invention relates in one aspect to a computer-readable medium, on which program code sections of a computer program are saved, the program code sections being loadable into and/or executable in a local transmission unit to make the local transmission unit execute the method according to an aspect of the invention when the program code sections are executed in the local transmission unit.

The realization of the invention by a computer program product and/or a computer-readable medium has the advantage that already existing allocation systems can be easily adopted by software updates in order to work as proposed by the invention.

The computer program product can be, for example, a computer program or comprise another element apart from the computer program. This other element can be hardware, for example a memory device, on which the computer program is stored, a hardware key for using the computer program and the like, and/or software, for example a documentation or a software key for using the computer program.

The present invention is further described hereinafter with reference to illustrated embodiments shown in the accompanying drawings, in which:
- FIG 1: illustrates a block diagram of a client-server architecture which provides geometric modeling of components representing different parts of a real world object, according to an embodiment.
- FIG 2: illustrates a block diagram of a local transmission unit in which an embodiment of a method for allocating medical data record to a human resource data record can be implemented.
- FIG 3: illustrates a block diagram of a security module, according to an embodiment.
- FIG 4: illustrates a flowchart of an embodiment of a method of allocating medical data record to human resource data record.
- FIG 5: illustrates a flowchart of an embodiment of a method of determining a matching human resource data record.
- FIG 6: illustrates a flowchart of another embodiment of a method of determining a matching human resource data record.
- FIG 7: illustrates a flowchart of an embodiment of a method of obtaining preferences from a set of human resources.
- FIG 8: illustrates a block diagram of a client-server architecture which provides geometric modeling of components involved in transfer of medical data record from one client to another, according to an embodiment.
- FIG 9: illustrates a block diagram of a client-server architecture which provides geometric modeling of components involved in transfer of medical data record from one client to another, according to another embodiment.
- FIG 10: illustrates a flowchart of an embodiment of a meth-od of transferring medical data record from one client device to another client device.

Hereinafter, embodiments for carrying out the present invention are described in detail. The various embodiments are described with reference to the drawings, wherein like reference numerals are used to refer to like elements throughout. In the following description, for purpose of explanation, numerous specific details are set forth in order to provide a thorough understanding of one or more embodiments. It may be evident that such embodiments may be practiced without these specific details.

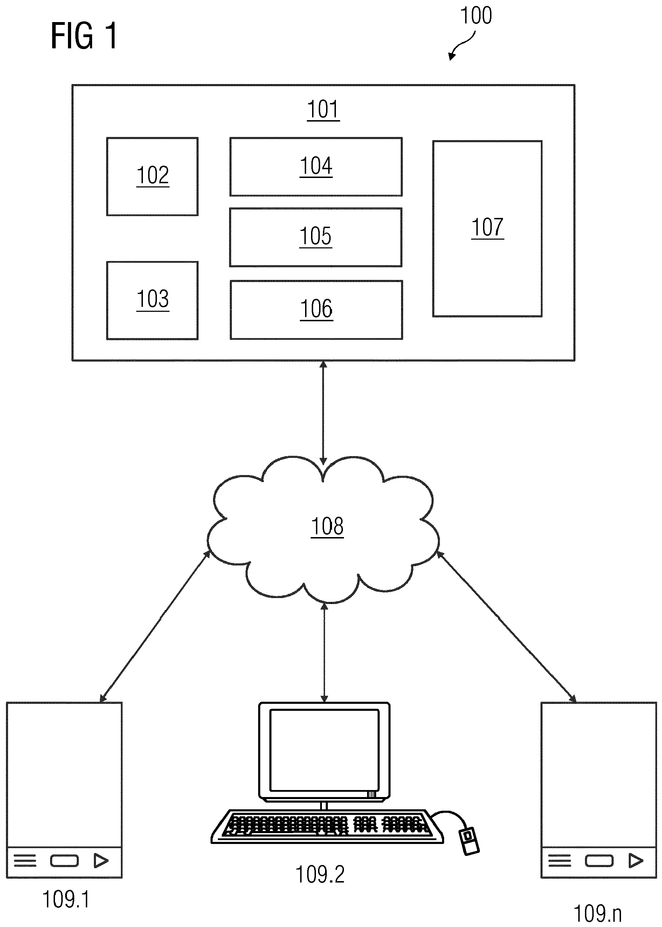

FIG 1 provides an illustration of a block diagram of a client-server architecture that is a geometric modelling of components representing different parts of real-world objects, according to an embodiment. The client-server architecture 100 includes a server 101 and a plurality of client devices 109.1-109.n. Each of the client devices 109.1-109.n are connected to the server 101 via a network 108, for example, local area network (LAN), wide area network (WAN), WiFi, etc. In one embodiment, the server 101 is deployed in a cloud computing environment. As used herein, "cloud computing environment" refers to a processing environment comprising configurable computing physical and logical resources, for example, networks, servers, storage, applications, services, etc., and data distributed over the network 108, for example, the in-ternet. The cloud computing environment provides on-demand network access to a shared pool of the configurable computing physical and logical resources. The server 101 may include a human resource preference database 102 that comprises human resource data records and preferences obtained from each one of a set of human resources. The server 101 may further include a medical database 103 that comprises medical data records of patients that are to be allocated to a human resource data record. The medical database may contain medical data obtained from, but not limited to, one or more medical equipments, for example scanners, hospitals, medical professionals, and other providers that may be involved in patient care. The medical database may obtain patient medical information from, for example, PACS (acronym for "Picture Archiving and Communication System"), HIS (acronym for "Hospital Information System"), LIS (acronym for "Laboratory Information System") and RIS (acronym for "Radiology Information System"). The server 101 may include a medical data analysis module 104 that performs analysis of the medical data record for allocation to a human resource data record. The server 101 also includes a match determination module 105 that determines a matching human resource data record for allocation of medical data record. The server 101 may also include a security module 106 that maintains the security of the medical data record. Additionally, the server 101 may include a network interface 107 for communicating with the client devices 109.1-109.n via the network 108.

The client devices 109.1-109.n includes a user device 109.1, used by a user. In an embodiment, the user device 109.1 may be used by a human resource to access allocated medical data record. The medical data record on the server 101 can be accessed by the user via a graphical user interface of an end user web application. In an embodiment, the human resource can analyze and make changes to the medical data record and save the updated medical data record on the server 101. In an embodiment, the human resource can access the medical data record on the server 101 via the network interface 107, on another client device 109.2. The human resource may access the medical data record via a graphical user interface. In another embodiment, the human resource may also send a request to the server 101 to share the medical data record with other specified entities via the network 108.

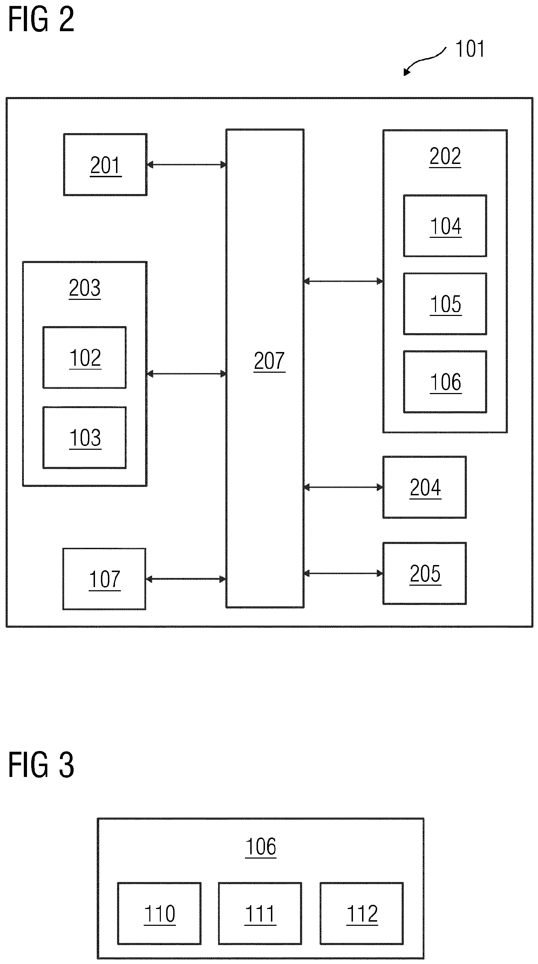

FIG 2 is a block diagram of a local transmission unit 101 in which an embodiment can be implemented, for example, as a system to allocate medical data record to human resource data record, configured to perform the processes as described therein. It is appreciated that the server 101 is an exemplary implementation of the local transmission unit in FIG 1. In FIG 2, the local transmission unit 101 comprises a processing unit 201, a memory 202, a storage unit 203, an input unit 204, an output unit 205 a network interface 107 and a standard interface or bus 207. The local transmission unit 101 can be a (personal) computer, a workstation, a virtual machine running on host hardware, a microcontroller, or an integrated circuit. As an alternative, the local transmission unit 101 can be a real or a virtual group of computers (the technical term for a real group of computers is "cluster", the technical term for a virtual group of computers is "cloud").

The processing unit 201, as used herein, means any type of computational circuit, such as, but not limited to, a microprocessor, microcontroller, complex instruction set computing microprocessor, reduced instruction set computing microprocessor, very long instruction word microprocessor, explicitly parallel instruction computing microprocessor, graphics processor, digital signal processor, or any other type of processing circuit. The processing unit 201 may also include embedded controllers, such as generic or programmable logic devices or arrays, application specific integrated circuits, single-chip computers, and the like. In general, a processing unit 201 can comprise hardware elements and software elements. The processing unit 201 can be configured for multi-threading, i.e. the processing unit 201 can host different calculation processes at the same time, executing the either in parallel or switching between active and passive calculation processes.

The memory 202 may be volatile memory and non-volatile memory. The memory 202 may be coupled for communication with the processing unit 201. The processing unit 201 may execute instructions and/or code stored in the memory 202. A variety of computer-readable storage media may be stored in and accessed from the memory 202. The memory 202 may include any suitable elements for storing data and machine-readable instructions, such as read only memory, random access memory, erasable programmable read only memory, electrically erasable programmable read only memory, a hard drive, a removable media drive for handling compact disks, digital video disks, diskettes, magnetic tape cartridges, memory cards, and the like. In the present embodiment, the memory 202 includes a medical data analysis module 104, a match determination module 105 and a security module 106 stored in the form of machine-readable instructions on any of the above-mentioned storage media and may be in communication to and executed by processing unit 201. When executed by the processing unit 201, the data analysis module 104 causes the processing unit 201 to analyze the medical data record and determine the medical parameters and recommendations related to data allocation. When executed by the processing unit 201, the match determination module 105 causes the processing unit 201 to determine a matching human resource data record based on defined preferences and allocate the medical data record to such matched human resource data record. When executed by the processing unit 201, the security module 105 causes the processing unit 201 to encrypt the medical data record and authenticate any request received to access to the medical data record. Method steps executed by the processing unit 201 to achieve the abovementioned functionality are elaborated upon in detail in Figure 3, 4, 5, 6, 8, 9 and 10.

The storage unit 203 may be a non-transitory storage medium which stores a medical database 102. The medical database 102 is a repository of medical information related to one or more patients that is maintained by a healthcare service provider. The storage unit 203 may also store a human resource preference database 103. The human resource preference database 103 is a repository of human resource data records and preferences obtained from each one of a set of human resources based on their availability, area of expertise and area of interest of such human resources. The input unit 204 may include input means such as keypad, touch-sensitive display, camera (such as a camera receiving gesture-based inputs), etc. capable of receiving input signal such as a medical data including patient information to be shared or allocated. The bus 207 acts as interconnect between the processing unit 201, the memory 202, the storage unit 203, the communication interface 107 the input unit 204 and the output unit 205.

Those of ordinary skilled in the art will appreciate that the hardware depicted in FIG 2 may vary for particular implementations. For example, other peripheral devices such as an optical disk drive and the like, Local Area Network (LAN)/ Wide Area Network (WAN)/ Wireless (e.g., Wi-Fi) adapter, graphics adapter, disk controller, input/output (I/O) adapter also may be used in addition or in place of the hardware depicted. The depicted example is provided for the purpose of explanation only and is not meant to imply architectural limitations with respect to the present disclosure.

A local transmission unit in accordance with an embodiment of the present disclosure includes an operating system employing a graphical user interface. The operating system permits multiple display windows to be presented in the graphical user interface simultaneously with each display window providing an interface to a different application or to a different instance of the same application. A cursor in the graphical user interface may be manipulated by a user through the pointing device. The position of the cursor may be changed and/or an event such as clicking a mouse button, generated to actuate a desired response.

One of various commercial operating systems, such as a version of Microsoft Windows™, a product of Microsoft Corporation located in Redmond, Washington may be employed if suitably modified. The operating system is modified or created in accordance with the present disclosure as described.

Disclosed embodiments provide systems and methods for analyzing medical data associated with a patient. In particular, the systems and methods may perform allocation of medical data to at least one human resource.

FIG 3 illustrates a block diagram of a security module 106. The security module 106 is configured to maintain the security of the medical data record to be allocated to a human resource data record. The security module 106 further comprises an encryption module 110, a decryption module 111 and an authentication module 112. The encryption module 110 is configured to encrypt the medical data record before the medical data record is allocated to any human resource. Therefore, the medical data record is stored in an encrypted format in the medical database 103. The encryption module 110 may perform encryption of the medical data record using an encryption algorithm that may be well known to person skilled in the art. The decryption module 111 decrypts the encrypted medical data record and makes the decrypted medical data available to the human resource. Such decryption of medical data is performed by a decryption algorithm and may also involve use of a key. The key may be a short string of characters which is needed to decrypt the data. The security module 106 further includes an authentication module 112 which digitally authenticates any request received by the server to access the medical data record. The authentication enables establishment of confidence for human resource identity. The authentication may be performed by the local transmission unit 101 using methods that may be well-known to a person skilled in the art.

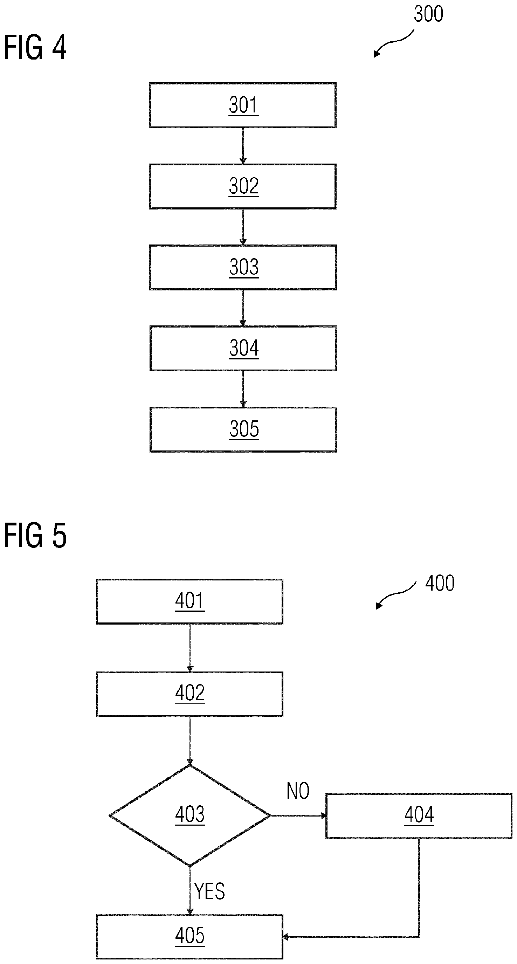

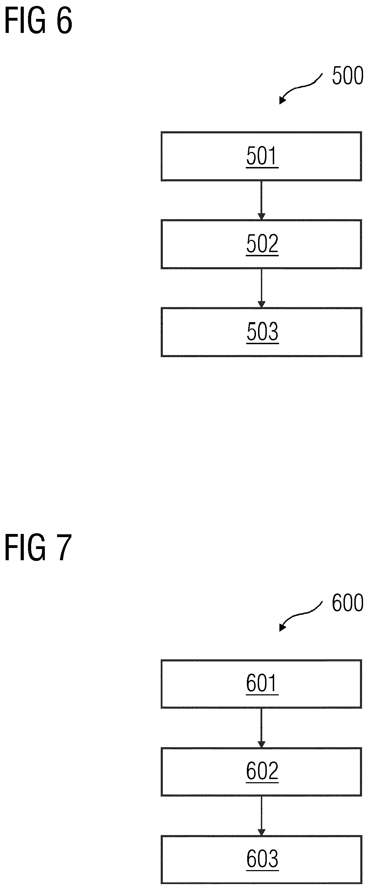

FIG 4 illustrates an embodiment of a method 300 of allocating medical data record to a human resource data record. At step 301 of the method 300 medical data record is received from a source through the standard interface 107. The source may be, for example, a medical database 103 containing a plurality of medical records. Such medical database 103 may be may be maintained, for example, by a hospital administration. The medical data record contains a first data item and a second data item. The first data item includes patient personal information and the second data item includes patient diagnosis information. The patient diagnosis information comprises medical parameters such as patient examination history, medical treatments and/or referrals. The patient personal information includes patient identification information and other personal data such as age, sex, and contact information. Patient examination information may include for example, but not limited to, one or more medical images, physical and chemical diagnostic tests values and results. At step 302, the medical data record is analyzed by the processing unit 201 to identify a medical parameter related to the medical data record. The medical data record may contain referrals which include a recommendation. Such recommendation may include information related to a human resource who may be eligible to analyze the medical data record. Advantageously, determination of the medical parameters of the medical data record enables efficient allocation of the medical data record to a human resource. Once the parameters are determined, at step 303, a preference defined by each one of a set of human resources is determined by the processing unit 201. The preference comprises a determinant defined by a human resource for allocation of the medical data record to the human resource data record. The preferences may include, for example, availability of the one or more human resources, area of expertise and area of interest of the one or more human resources. FIG 7 illustrates a flowchart of a method 600 of obtaining the preferences from each one of the set of human resources. In order to determine the preferences, a questionnaire may be provided to the human resources, at step 601. The human resources may be able to access the questionnaire and submit the responses to the questionnaire through a graphical user interface of an end-user web application. At step 602, the responses are obtained from the human resources. The questionnaire may comprise, for example, multiple choice questions. In an embodiment, the graphical user interface also includes one or more radio buttons against the options for the questions, which may be clicked using a mouse button to choose the response. Responses obtained to the questionnaire are used to determine factors such as availability, the area of expertise and areas of interest of the human resources. In the embodiment, the graphical user interface includes an option to save the preferences specified by the one or more human resources. In the embodiment, the option to save may be included in the graphical user interface in the form of a button that can be clicked on by a mouse so as to trigger an action for saving the preferences. The saved preferences are stored in the human resource preference database 102, at step 603.

At step 304, a matching human resource is determined by the processing unit 201 based on the determined parameters of the medical data. FIG 6 illustrates an embodiment of a method 500 of determining a matching human resource data record. At step 502, the preferences are analyzed taking into consideration the determined parameters of the medical data record. The medical parameters are mapped to the preference to determine a matching human resource for analysis of the medical data. In order to determine the matching human resource, the availability, area of expertise and defined area of interest of each one of the set of human resources are examined. The preferences are mapped to the parameters determined from the medical data record. One or more human resources may initially be shortlisted, for example, based on the area of expertise. The area of expertise of the human resources may be similar to a medical field in which the diagnosis information of a patient may lie. The area of interest of the human resources may be used, for example, to narrow down further on the eligible human resource. The closest matching human resource may be determined, for example, considering the availability of the human resources to analyze the medical data record. For example, if two human resources are eligible to analyze the medical data record, the matching human resource out of the eligible two human resources is identified depending on the schedule of the human resources. On determination of the matching human resource, the medical data is allocated to the human data record of the matching human resource, at step 305 and 503. In an embodiment, the matching human resource may be notified of such allocation of the medical data record. The notification may be, for example, performed using push notifications on a web-based profile on the end-user web application. Alternatively, the matching human resource may be notified of allocation of the medical data record through a text messaging service on a mobile device.

FIG 5 illustrates a flowchart of another embodiment of a method 400 of determining a matching human resource for allocation of medical data record. At step 401, the medical data record to be allocated is received from the medical database 103. At step 402, the medical data record is analyzed to extract the second data item. The second data item includes patient diagnosis information, for example, medical examination history, medical treatments and/or referrals. The referrals may include a recommendation for a human resource that may be eligible to analyze the medical data record. Based on the recommendation, if a human resource has been included in the medical data record is determined. If the human resource has been included in the medical data record, at step 404, the medical data record is allocated to the human resource data record of the human resource. If no human resource has been included in the medical data record, a matching human resource is determined as described in method 500.

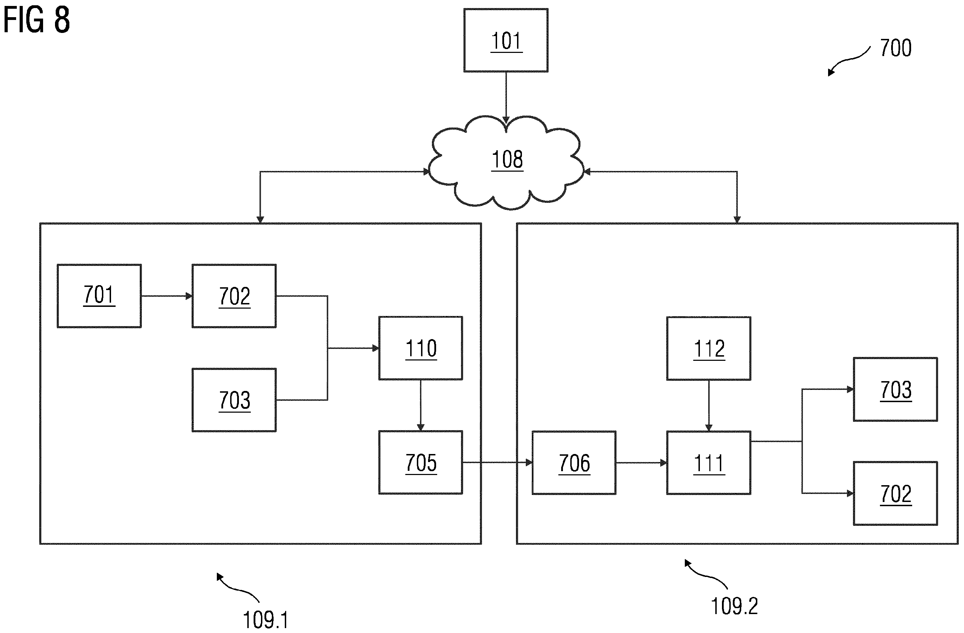

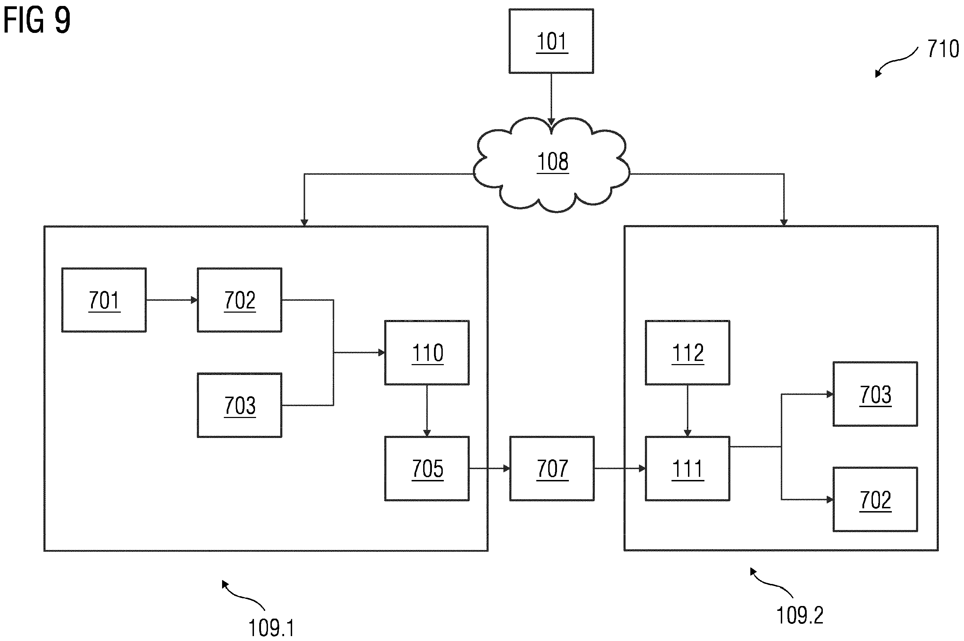

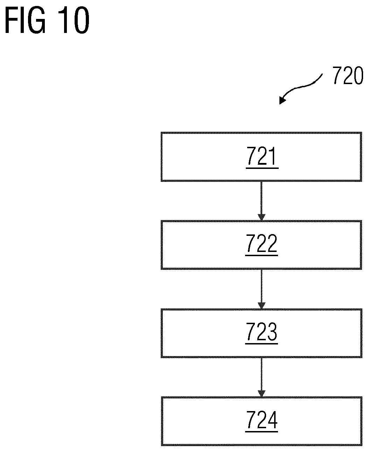

FIG 8 illustrates a block diagram of a client-server architecture 700 which provides geometric modeling of components involved in transfer of medical data record from one client to another, according to an embodiment. In the embodiment, the client devices include a computing unit 109.1 and a mobile device 109.2. FIG 10 illustrates an embodiment of a method 720 of transferring medical data record from one client device to another client device. The computing unit 109.1 includes an app-state ID generator 701. The app-state ID generator generates an app-state ID 702, at step 721. The app-state ID enables identification of an app-state. An app-state refers to a state which the medical data record is in. Therefore, if a human resource makes any change to the medical data record or updates the medical data record, the app-state indicates the current/present state in which the medical data record exists. The medical data record and the app-state of the medical data record 703 is encrypted along with app-state ID 702 using an encryption module 110, at step 722. The encryption module 110 creates an encoded image 705 for transfer of the medical data record from the computing unit 109.1 to the mobile device 109.2. At step 723, the encoded image is transferred to the mobile device 109.2. The encoded image 705 is recognized using, for example, an embedded camera apparatus 706 of the mobile device 109.2. The mobile device 109.2 includes the decryption module 111 which decrypts the encoded image 705 recognized by the camera apparatus 706. The mobile device 109.2 also includes the authentication module 112 which authenticates the human resource before access to the medical data record is provided. The authentication enables establishment of confidence for human resource identity. The authentication may be performed using methods that may be well-known to a person skilled in the art. On successful authentication, the encrypted medical data record and app-state 703 along with the app-state ID 702 is decrypted, at step 724, and is displayed to the human resource. An alternate embodiment of sharing the medical data record between two client devices is illustrated using a client server architecture 710, in FIG 9. In the embodiment, the first client device 109.1 encrypts the medical data record with app-state and app-ID using the encryption module 110 to create an encoded data 705. The encoded data 705 is embedded in a web-based link and is transferred to the second client device 109.2 via an email. Such transference of medical data record along with the app-state enables the human resource to continue the analysis of the medical data on more than one device. Alternatively, the matched human resource may also share the medical data record with a second human resource, for example, to obtain a second opinion.

The foregoing examples have been provided merely for the purpose of explanation and are in no way to be construed as limiting of the present invention disclosed herein. While the invention has been described with reference to various embodiments, it is understood that the words, which have been used herein, are words of description and illustration, rather than words of limitation. Further, although the invention has been described herein with reference to particular means, materials, and embodiments, the invention is not intended to be limited to the particulars disclosed herein; rather, the invention extends to all functionally equivalent structures, methods and uses, such as are within the scope of the appended claims. Those skilled in the art, having the benefit of the teachings of this specification, may effect numerous modifications thereto and changes may be made without departing from the scope and spirit of the invention in its aspects.

## Claims

1. A method of allocating a medical data record to a matching human resource data record, the method comprising:
receiving (301) a medical data record from a source (103) by an interface (207);
receiving (301) a set of human resource data records from a second source (104) by the interface (207);
analyzing (302) the medical data record by a processing unit (201) to identify a medical parameter of the medical data record;
determining (303) a preference for each one of the set of human resource data records by the processing unit (201);
determining (304) a matching human resource data record from the set of human resource data records based on the preference and the parameter of the medical data record by the processing unit (201); and
allocating (305) the medical data record to the matched human resource data record by the processing unit (201).

2. The method according to claim 1, wherein the preference comprises a determinant defined by a human resource for allocation of the medical data record to the human resource data record.

3. The method according to claim 1 and 2, wherein the preference is chosen from a group comprising area of specialization of the human resource, availability of the human resource, and area of interest of the human resource.

4. The method according to one of the preceding claims,
wherein the medical data record comprises a first data item and a second data item,
wherein the first data item comprises personal data related to a patient,
wherein the second data item comprises patient diagnosis information.

5. The method according to claim 1, wherein in analyzing the medical data record, the method comprises:
extracting (402) the second data item from the medical data record by the processing unit (201); and
determining (403) from the second data item the medical parameter by the processing unit (201).

6. The method according to one of the preceding claims, wherein the parameter of the medical data record comprises medical examination history, medical treatment and/or referrals documented in the medical data record.

7. The method according to claim 1 and 5, wherein in determining a matching human resource data record, the method comprises:
determining (403) from the second data item whether a human resource has been specified in the medical data record by the processing unit (201);
if a human resource is specified, allocating (405) the medical data record by the processing unit (201) to the human resource data record of the specified human resource; and
if no human resource is specified, determining (404) a matching human resource by the processing unit (201).

8. The method according to claim 7, wherein in determining a matching human resource data record, the method comprises:
analyzing (502) the preference determined for each one of the set of human resources data records to determine a human resource whose preference matches with the medical parameter by the processing unit (201); and
allocating (503) the medical data record to the human resource data record of the matched human resource by the processing unit (201).

9. The method according to one of the preceding claims, further comprising encrypting (723) the medical data record by the processing unit (201) before allocating the medical data record to the matched human resource data record.

10. The method according to claim 9, further comprising:
receiving from a server a request to access the encrypted medical data record;
authenticating the request to identify if the request is received from an authentic human resource by the processing unit (201); and
sharing decrypted medical data record with the human resource by the processing unit (201), if such a request is authenticated.

11. The method according to one of the preceding claims, wherein the human resource can share the medical data record with one or more human resources.

12. A local transmission unit (101) for allocating a medical data record to a matching human resource data record, the local transmission unit (101) comprising:
an interface (207) configured to receive (301) a medical data record from a source (103),
furthermore configured to receive (301) a set of human resource data records from a second source (104); and
a processing unit (201) configured to analyze (302) the medical data record to identify a parameter of the medical data record,
furthermore configured to determine (303) a preference for each one of the set of human resource data records, furthermore configured to determine (304) a matching human resource data record from the set of human resource data records based on the preference and the parameter of the medical data record, and
furthermore configured to allocate (305) the medical data record to the matched human resource data record.

13. The local transmission unit (101) according to claim 12, furthermore configured to execute a method according to one of the claims 2 to 11.

14. A computer program product comprising a computer program, the computer program being loadable into a storage unit (203) of a local transmission unit (101), including program code sections to make the local transmission unit (101) execute the method of any one of claims 1 to 11 when the computer program is executed in the local transmission unit (101).

15. A computer-readable medium, on which program code sections of a computer program are saved, the program code sections being loadable into and/or executable in an allocation system to make the local transmission unit (101) execute the method of any one of the claims 1 to 11 when the program code sections are executed in the local transmission unit (101).
